# EUROPÄISCHE PATENTSCHRIFT

(11) **EP 3 160 537 B1**
(45) Veröffentlichungstag und Bekanntmachung des Hinweises auf die Patenterteilung: **19.09.2018**
(21) Anmeldenummer: 15729488.5
(22) Anmeldetag: 17.06.2015
(51) Int. Cl.: A61M 1/36, G08B 21/00, H04B 7/00

(54) **VORRICHTUNG ZUR ÜBERWACHUNG EINES GEFÄSSZUGANGS FÜR EINE EXTRAKORPORALE BLUTBEHANDLUNGSVORRICHTUNG UND VERFAHREN ZUR ÜBERWACHUNG EINES GEFÄSSZUGANGS**
DEVICE FOR MONITORING A VESSEL OPENING FOR AN EXTRACORPOREAL BLOOD TREATMENT DEVICE AND METHOD FOR MONITORING A VESSEL OPENING
DISPOSITIF DE SURVEILLANCE D'UN ACCÈS VASCULAIRE POUR UN DISPOSITIF DE TRAITEMENT EXTRACORPOREL DU SANG ET PROCÉDÉ DE SURVEILLANCE D'UN ACCÈS VASCULAIRE

(30) Priorität: 25.06.2014 DE 102014009388
(43) Veröffentlichungstag der Anmeldung: 03.05.2017
(73) Patentinhaber: Fresenius Medical Care Deutschland GmbH, 61352 Bad Homburg (DE)
(72) Erfinder: STAHL, Thomas, 97839 Esselbach (DE)
(74) Vertreter: Oppermann, Frank
(86) Internationale Anmeldenummer: PCT/EP2015/063619
(87) Internationale Veröffentlichungsnummer: WO 2015/197450

(56) Entgegenhaltungen:
- EP-A2- 2 331 212
- WO-A1-2011/116943
- US-A1- 2005 038 325
- US-A1- 2014 100 519
- US-B1- 8 398 575

## Beschreibung

Die Erfindung betrifft eine Vorrichtung zur Überwachung eines Gefäßzugangs für eine extrakorporale Blutbehandlungsvorrichtung mit einem extrakorporalen Blutkreislauf, der eine arterielle Blutleitung mit einer arteriellen Kanüle und eine venöse Blutleitung mit einer venösen Kanüle aufweist. Darüber hinaus betrifft die Erfindung eine Anordnung mit einer Vorrichtung zur Überwachung eines Gefäßzugangs und einer extrakorporalen Blutbehandlungsvorrichtung sowie ein Verfahren zur Überwachung eines Gefäßzugangs während einer extrakorporalen Blutbehandlung.

Bei der extrakorporalen Blutbehandlung wird dem Patienten über eine arterielle Schlauchleitung mit einer arteriellen Kanüle Blut entnommen, das dem Patienten über eine venöse Schlauchleitung mit einer venösen Kanüle wieder zugeführt wird. Unter einer Kanüle kann beispielsweise eine Punktionskanüle, Dialysenadel oder Nadel verstanden werden. Die extrakorporalen Blutbehandlungsvorrichtungen, die über einen extrakorporalen Blutkreislauf verfügen, setzen einen ordnungsgemäßen Zugang zu dem Patienten voraus. Zu den bekannten extrakorporalen Blutbehandlungsvorrichtungen zählen beispielsweise Dialysevorrichtungen und Zellseparatoren.

Obwohl der Gefäßzugang bei der Blutbehandlung laufend überwacht wird, besteht grundsätzlich die Gefahr, dass die Kanüle unbemerkt aus dem Blutgefäß des Patienten herausrutscht. Zur Überwachung des Gefäßzugangs sind verschiedene Vorrichtungen unterschiedlicher Ausbildung bekannt, die bei einem nicht ordnungsgemäßen Gefäßzugang eine sofortige Unterbrechung des extrakorporalen Blutkreislaufs auslösen.

Aus der WO 2011/116943 A1 ist eine Vorrichtung zur Überwachung eines Gefäßzugangs bekannt, die über einen Sensor verfügt, der eine für den Zustand des Gefäßzugangs charakteristische Größe erfasst. Der Sensor ist als ein Pad ausgebildet, das an der Punktionsstelle auf die Haut des Patienten aufgelegt wird. Als für den Zustand des Gefäßzugangs charakteristische Größe misst der Sensor an der Punktionsstelle die Feuchtigkeit oder das Austreten von Flüssigkeit. Der Sensor ist über ein Verbindungskabel mit einer Auswerteinheit verbunden, mit der das Sensorsignal ausgewertet wird. Wenn Blut an der Punktionsstelle austritt, wird ein akustischer Alarm gegeben und die Blutbehandlung unterbrochen.

Eine Vorrichtung zur Überwachung eines Gefäßzugangs während der Dialyse ist auch aus der US 2005/0038325 A1 bekannt. Die Überwachungsvorrichtung verfügt über einen Feuchtigkeitssensor, der über ein Verbindungskabel mit einer Auswerteinheit verbunden ist, die einen Alarmgeber aufweist, der bei einem fehlerhaften Gefäßzugang einen Vibrationsalarm gibt.

Die US 8,398,575 B1 beschreibt ebenfalls eine Überwachungsvorrichtung mit einem Feuchtigkeitssensor und einer Auswerteinheit, die über ein Kabel miteinander verbunden sind. Die Auswerteinheit gibt bei einem fehlerhaften Gefäßzugang auch einen Vibrationsalarm. Auch die US 8 348 850 B2 schlägt vor, bei einem fehlerhaften Gefäßzugang einen Vibrationsalarm zu geben, so dass der Patient, wenn er schlafen sollte, geweckt wird.

Ein kabelloses medizinisches Diagnose- und Überwachungsgerät, das einen Sensor und eine Auswerteinheit aufweist, ist aus der DE 43 29 898 A1 bekannt. Der Sensor und die Auswerteinheit weisen jeweils eine Datenübertragungseinheit auf, zwischen denen eine Übertragung von Funksignalen erfolgt, wobei die Sendeleistung automatisch an die örtlichen Gegebenheiten angepasst wird. Zum Schutz des Patienten vor einer zu hohen elektromagnetischen Strahlung wird die Funkverbindung überwacht. Bei einer zu hohen Sendeleistung wird die Funkverbindung unterbrochen und Alarm gegeben.

Der Erfindung liegt die Aufgabe zugrunde, eine nicht über ein Kabel an die extrakorporale Blutbehandlungsvorrichtung gebundene Vorrichtung zur Überwachung eines Gefäßzugangs zu schaffen, die zusammen mit der Blutbehandlungsvorrichtung eine Überwachung des Gefäßzugangs mit hoher Sicherheit erlaubt. Darüber hinaus liegt der Erfindung die Aufgabe zugrunde, ein Verfahren zur Überwachung eines Gefäßzugangs während einer extrakorporalen Blutbehandlung anzugeben, das eine Überwachung des Gefäßzugangs zusammen mit der Blutbehandlungsvorrichtung auch dann mit hoher Sicherheit erlaubt, wenn die Vorrichtung zur Überwachung eines Gefäßzugangs nicht über ein Kabel an die Blutbehandlungsvorrichtung gebunden ist.

Die Lösung dieser Aufgaben erfolgt mit den Merkmalen der unabhängigen Ansprüche. Die abhängigen Ansprüche betreffen vorteilhafte Ausführungsformen der Erfindung.

Die erfindungsgemäße Vorrichtung zur Überwachung eines Gefäßzugangs für eine extrakorporale Blutbehandlungsvorrichtung verfügt über einen Sensor zur Erfassung von mindestens einer physikalischen Größe, die für den Zustand des Gefäßzugang charakteristisch ist, und ein mit dem Sensor über ein Verbindungskabel verbundenes Auswertgerät, das eine Auswerteinheit aufweist. Die Auswerteinheit, die die gemessene physikalische Größe auswertet, erzeugt für den Zustand des Gefäßzugangs charakteristischer Signale.

Die für den Zustand des Gefäßzugangs charakteristischer Signale können Signale sein, die einen fehlerfreien und/oder fehlerhaften Gefäßzustand, d.h. den ordnungsgemäßen Sitz der Kanüle oder das Herausrutschen der Kanüle signalisieren. Insbesondere ist das Signal ein Signal, das einen fehlerhaften Zustand signalisiert.

Der Sensor wird an der Punktionsstelle befestigt, wobei das kabelgebundene Auswertgerät von der Punktionsstelle entfernt angeordnet wird. Das Auswertgerät weist eine Datenübertragungseinheit zur Herstellung einer drahtlosen Verbindung zwischen dem Auswertgerät und der Blutbehandlungsvorrichtung auf, so dass das Auswertgerät nicht über ein Kabel an die Behandlungsvorrichtung gebunden ist. Die drahtlose Verbindung zwischen Auswertgerät und Blutbehandlungsvorrichtung kann eine Verbindung über Funksignale oder optische Signale sein. Wenn die drahtlose Verbindung über Funksignale erfolgt, sollte die Sendeleistung zum Schutz des Patienten vor übermäßiger elektromagnetischer Strahlung und/oder zur Verringerung des Energieverbrauchs für eine Verlängerung der Betriebszeit bei Batterie- oder Akku-Betrieb gering sein. Blutbehandlungsvorrichtung und Auswertgerät sind einander eindeutig zugeordnet, beispielsweise durch eine Bestätigung einer korrekten Zuordnung durch das Bedienpersonal innerhalb eines vorgegebenen Zeitintervalls. Bei geringer Sendeleistung besteht die drahtlose Verbindung nur im Nahbereich des Auswertgerätes, so dass eine Verbindung zu anderen Blutbehandlungsvorrichtungen auf der Dialysestation, die sich nicht im Nahbereich des Auswertgerätes befinden, auch nicht aufgebaut werden könnte.

Angesicht der relativ niedrigen Sendeleistung kann die drahtlose Verbindung zwischen dem Auswertgerät und der Blutbehandlungsvorrichtung während einer Blutbehandlung dadurch unterbrochen werden, dass sich der Patient auf das Auswertgerät legt. Wenn der Patient während der Blutbehandlung eingeschlafen sein sollte, wird er die Unterbrechung der Funkverbindung nicht bemerken, so dass beim Herausrutschen der Kanüle die Blutbehandlung nicht unterbrochen und kein Alarm gegeben wird.

Das Auswertgerät der erfindungsgemäßen Überwachungsvorrichtung zeichnet sich durch eine Kontrolleinheit aus, die derart konfiguriert ist, dass eine Unterbrechung der drahtlosen Verbindung zwischen dem Auswertgerät und der Blutbehandlungsvorrichtung erkannt wird. Darüber hinaus weist die Überwachungsvorrichtung eine Signaleinheit auf, die ein Signal gibt, wenn die Kontrolleinheit die Unterbrechung der drahtlosen Verbindung erkennt. Die Signaleinheit zeichnet sich durch einen Vibrations-Signalgeber aus, der die Unterbrechung der drahtlosen Verbindung mit Vibrationen signalisiert. Die sich auf den Körper des Patienten, der auf dem Auswertgerät liegt, übertragenden Vibrationen führen dazu, dass der Patient aufgeweckt wird, so dass er sich bewegen und das Auswertgerät freigeben kann. Diese Signalisierung einer gestörten Verbindung zwischen den Geräten ist von dem eigentlichen Alarm zu unterscheiden, der ausgelöst wird, wenn ein fehlerhafter Gefäßzugang festgestellt wird. Ein Alarm richtet sich insbesondere an das medizinische Personal, das geeignete Gegenmaßnahmen ergreifen soll, um die Blutbehandlungsvorrichtung in einen für den Patienten sicheren Zustand zu überführen, beispielsweise durch Stoppender Blutpumpe und/oder Schließen der venösen und/oder arteriellen Schlauchklemme.

Eine bevorzugte Ausführungsform der Erfindung sieht vor, dass die Kontrolleinheit derart konfiguriert ist, dass auf eine Unterbrechung der drahtlosen Verbindung zwischen dem Auswertgerät und der Blutbehandlungsvorrichtung erst dann geschlossen wird, wenn die drahtlose Verbindung für eine Zeitdauer unterbrochen ist, die länger als ein vorgegebener Zeitraum ist. Dadurch soll erreicht werden, dass nur kurzzeitige Lageänderungen des Patienten nicht gleich zu einer Signalisierung der Unterbrechung der drahtlosen Verbindung durch Vibrationen führen.

Bei einer bevorzugten Ausführungsform werden die für den Zustand des Gefäßzugangs charakteristischen Signale mit einer Sendeeinheit an die Blutbehandlungsvorrichtung gesendet. Für die Übertragung der für die Überwachung des Gefäßzugangs relevanten Daten ist eine unidirektionale Verbindung ausreichend. Die bevorzugte Ausführungsform sieht neben der Sendeeinheit eine Empfangseinheit vor, mit der zur Überwachung der drahtlosen Verbindung von der Blutbehandlungsvorrichtung Bestätigungssignale empfangen werden. Die Kontrolleinheit ist vorzugsweise derart konfiguriert, dass sie erkennt, wenn die Empfangseinheit ein das Bestehen der drahtlosen Verbindung signalisierendes Bestätigungssignal der Blutbehandlungsvorrichtung nicht empfängt. Da das Auswertgerät zumindest in vorgegebenen Zeitintervallen die für den Zustand des Gefäßzugangs charakteristischen Signale an die Blutbehandlungsvorrichtung sendet, kann auf eine Unterbrechung der drahtlosen Verbindung auch dann geschlossen werden, wenn die Blutbehandlungsvorrichtung diese Signale nicht empfängt. Dann kann die Blutbehandlungsvorrichtung ein die Unterbrechung der drahtlosen Verbindung signalisierendes Signal an das Auswertgerät senden.

Für die Erfindung ist unerheblich, wie der Sensor ausgebildet ist und auf welchem Funktionsprinzip der Sensor beruht. Der Sensor ist bei einer bevorzugten Ausführungsform als ein auf die Haut des Patienten aufzulegendes Pad aus einem flexiblen Material ausgebildet. Vorzugsweise weist der Sensor als Sensor-Element einen Feuchtigkeits- oder Flüssigkeitssensor auf, mit dem das Austreten von Blut an der Punktionsstelle festgestellt werden kann, wenn die Kanüle herausgerutscht sein sollte.

Die Vibrationen sollten ausreichend stark sein, so dass der Patient auch aus einem tiefen Schlaf aufgeweckt wird. Zur Erzeugung eines möglichst starken Vibrationssignals ist der Vibrations-Signalgeber vorzugsweise mit einer Wand des Gehäuses des Auswertgerätes verbunden, auf das sich die Vibrationen übertragen sollen. Das Gehäuse ist vorzugsweise ein als ein flaches Gehäuse mit einer Vorderwand und einer Rückwand und einer schmalen Seitenwand ausgebildet, wobei der Vibrations-Signalgeber mit der Vorderwand und/oder Rückwand verbunden ist und das Verbindungskabel an der Seitenwand aus dem Gehäuse herausgeführt ist. Wenn sich der Patient auf das Auswertgerät legt, wird sein Körper auf der vorderen oder hinteren flachen Gehäusewand zu liegen kommen. Aufgrund der relativ großen Anlagefläche wird der Patient die Vibrationen deutlich spüren.

Die extrakorporale Blutbehandlungsvorrichtung weist eine Datenübertragungseinheit zur Herstellung einer drahtlosen Verbindung zwischen der Blutbehandlungsvorrichtung und dem Auswertgerät auf, wobei die Datenübertragungseinheit des Auswertgerätes und der Blutbehandlungsvorrichtung eine Schnittstelle bilden, die auf den unterschiedlichen Standards beruhen kann. Beispielsweise kann die Schnittstelle nach dem sogenannten Bluetooth-Standard arbeiten. Die Schnittstelle könnte aber auch eine mit optischen Signalen arbeitende Schnittstelle sein, die aber weniger geeignet als eine Funksignale übertragende Schnittstelle ist. Die bekannten Schnittstellen sehen entsprechende Sende- und Empfangseinheiten vor, über die eine Datenkommunikation stattfindet.

Die Blutbehandlungsvorrichtung empfängt die für den Zustand des Gefäßzugangs charakteristischen Signale, insbesondere ein Signal für einen fehlerhaften Gefäßzugang. Bei einer bevorzugten Ausführungsform weist die Blutbehandlungsvorrichtung eine Alarmeinheit auf, die derart ausgebildet ist, dass nach dem Empfangen eines für einen fehlerhaften Gefäßzugang charakteristischen Signals ein akustischer und/oder optischer Alarm erzeugt wird. Über eine derartige Alarmeinheit kann aber auch das Auswertgerät verfügen. Die Blutbehandlungsvorrichtung kann auch eine Signaleinheit aufweisen, die derart ausgebildet ist, dass nach der Unterbrechung der drahtlosen Verbindung ein akustisches und/oder optisches Signal erzeugt wird, so dass auch dem medizinischen Personal eine Unterbrechung der drahtlosen Verbindung signalisiert wird.

Die Signalisierung der Unterbrechung der drahtlosen Verbindung durch die Blutbehandlungsvorrichtung kann zeitversetzt zu der Signalisierung durch das Auswertgerät erfolgen. Vorzugsweise gibt zunächst das Auswertgerät ein Vibrations-Signal, so dass der Patient die drahtlose Verbindung wiederherstellen kann. Die Blutbehandlungsvorrichtung signalisiert die Unterbrechung der Verbindung vorzugsweise erst dann, wenn dem Patienten die Wiederherstellung der Verbindung innerhalb eines vorgegebenen Zeitintervalls nicht gelingen sollte. Dadurch wird das medizinische Personal entlastet. Es ist aber auch möglich, dass die Blutbehandlungsvorrichtung und das Auswertgerät die Unterbrechung der Verbindung zeitgleich signalisieren. Vorzugsweise sollte sich Blutbehandlungsvorrichtung und Auswertgerät derart konfigurieren lassen, dass sich die zeitliche Verzögerung, mit der die Blutbehandlungsvorrichtung auf die Unterbrechung der Verbindung reagiert, vom medizinischen Personal vorgeben lässt.

Im Folgenden wird ein Ausführungsbeispiel der Erfindung unter Bezugnahme auf die Figuren im Einzelnen erläutert.

Es zeigen:
- Fig. 1: die erfindungsgemäße Vorrichtung zur Überwachung eines Gefäßzugangs für eine Blutbehandlungsvorrichtung in der Draufsicht und
- Fig. 2: die wesentlichen Komponenten der Vorrichtung zur Überwachung eines Gefäßzugangs und einer Blutbehandlungsvorrichtung in schematischer Darstellung.

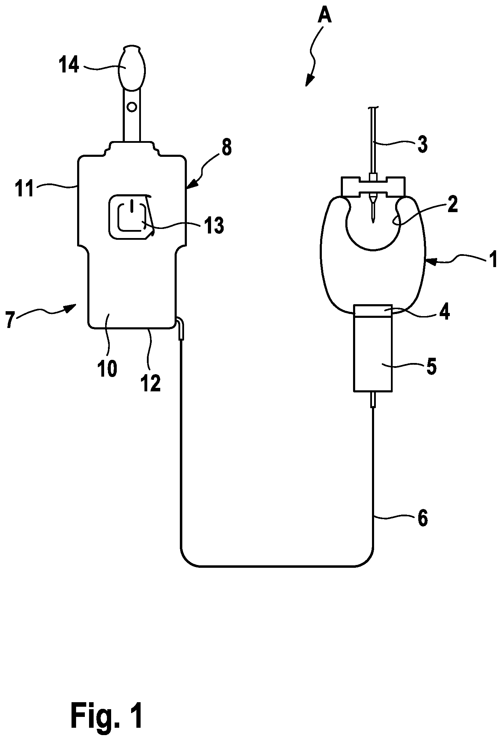

Fig. 1 zeigt ein Ausführungsbeispiel der erfindungsgemäßen Vorrichtung A zur Überwachung eines Gefäßzugangs für eine Blutbehandlungsvorrichtung. Die Überwachungsvorrichtung A weist einen Sensor 1 auf, der an der Punktionsstelle auf der Haut des Patienten befestigt werden kann. Der Sensor 1 ist als ein Pad aus einen flexiblen und saugfähigen Material ausgebildet, das an einer Seite einen Ausschnitt 2 zum Durchführen der Kanüle 3 aufweist. An der dem Ausschnitt gegenüberliegenden Seite weist der Sensor eine Anschlusslasche 4 für eine Anschlussklemme 5 eines Verbindungskabels 6 auf, das zu einem Auswertgerät 7 führt.

Der Sensor 1 weist ein nicht dargestelltes Sensor-Element auf, das eine für den Zustand des Gefäßzugangs charakteristisches Größe erfasst, die beispielsweise der elektrische Widerstand sein kann, der sich ändert, wenn das Pad mit Blut in Kontakt kommt. Zum Messen des elektrischen Widerstands kann das Sensor-Element beispielsweise eine elektrische Leiterschleife sein.

Das Auswertgerät 7 weist ein flaches Gehäuse 8 auf, das eine Vorderwand 10 und eine in Fig. 1 nicht sichtbare Rückwand 11 und eine schmale Seitenwand 12 hat. Auf der Vorderwand 10 befindet sich ein Bedienelement 13. Das zu dem Sensor führende Verbindungskabel 6 ist an der unteren schmalen Seitenwand des Gehäuses 8 herausgeführt. An der oberen Schmalseite des Gehäuses befindet sich ein Befestigungsclip 14, mit dem das Auswertgerät 7 beispielsweise an der Kleidung des Patienten befestigt werden kann.

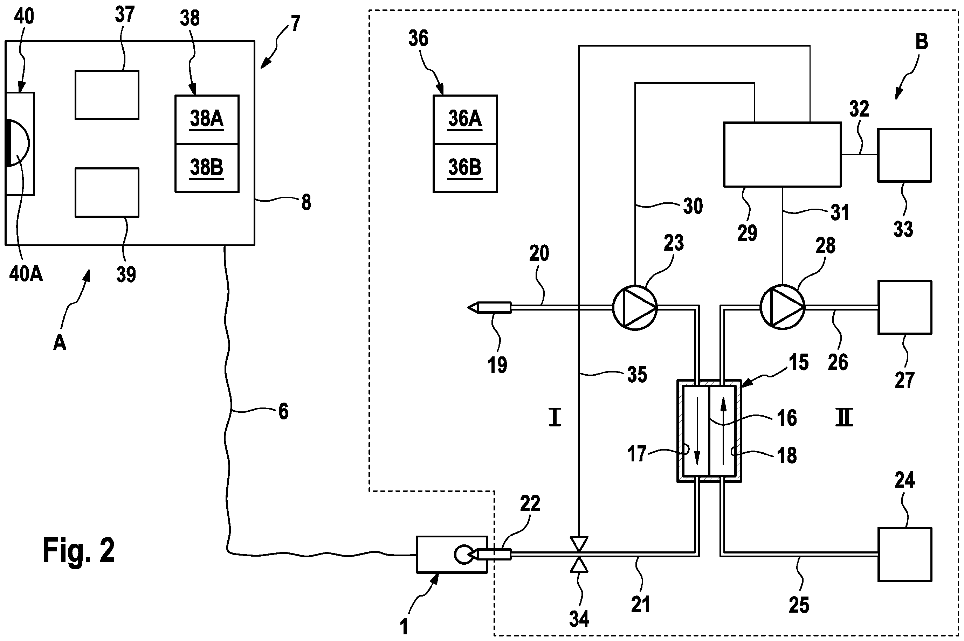

Das Auswertgerät 7 und der Sensor 1 der Überwachungsvorrichtung A bilden eine eigenständige Einheit, die über eine drahtlose Verbindung mit der Blutbehandlungsvorrichtung kommuniziert. Nachfolgend wird die Überwachungsvorrichtung A zusammen mit der Blutbehandlungsvorrichtung B unter Bezugnahme auf Fig. 2 beschrieben, in der die wesentlichen Komponenten der beiden Vorrichtungen schematisch dargestellt sind.

Die Blutbehandlungsvorrichtung B ist bei dem Ausführungsbeispiel eine Hämodialysevorrichtung, die einen Dialysator 15 aufweist, der durch eine semipermeable Membran 16 in eine Blutkammer 17 und eine Dialysierflüssigkeitskammer 18 unterteilt ist. An der nicht dargestellten Fistel oder dem Shunt des Patienten ist mittels einer arteriellen Kanüle 19 eine arterielle Schlauchleitung 20 angeschlossen, die zu dem Einlass der Blutkammer 17 des Dialysators 15 führt. Von dem Auslass der Blutkammer 17 des Dialysators 15 geht eine venöse Schlauchleitung 21 ab, die mittels einer venösen Kanüle 22 an der Fistel oder dem Shunt des Patienten angeschlossen ist. In die arterielle Schlauchleitung 20 ist in eine Blutpumpe 23 geschaltet, die das Blut im extrakorporalen Blutkreislauf I fördert.

Der Dialysierflüssigkeitskreislauf II der Dialysevorrichtung umfasst eine Dialyseflüssigkeitsquelle 24, an der eine Dialysierflüssigkeitszuführleitung 25 angeschlossen ist, die zu dem Einlass der Dialysierflüssigkeitskammer 18 des Dialysators 15 führt. Von dem Auslass der Dialysierflüssigkeitskammer 18 des Dialysators 15 geht eine Dialysierflüssigkeitsabführleitung 26 ab, die zu einem Auslass 27 führt. In die Dialysierflüssigkeitsabführleitung 26 ist eine Dialysierflüssigkeitspumpe 28 geschaltet.

Die Steuerung der Dialysevorrichtung übernimmt eine zentrale Steuereinheit 29, die über Steuerleitungen 30, 31 die Blut- und Dialysierflüssigkeitspumpe 23, 28 ansteuert. Die zentrale Steuereinheit 29 ist über eine Datenleitung 32 mit einer Alarmeinheit 33 verbunden, die bei einem Störfall einen optischen und/oder akustischen Alarm gibt.

Stromab der Blutkammer 17 des Dialysators 15 befindet sich an der venösen Schlauchleitung 21 eine elektromagnetisch betätigbare Schlauchklemme 34, die über eine weitere Steuerleitung 35 von der zentralen Steuereinheit 29 geschlossen wird, wenn die venöse Kanüle 22 (Nadel) aus dem Gefäßzugang herausrutschen sollte. Darüber hinaus stoppt die Steuereinheit 29 nach dem Herausrutschen der Kanüle die Blutpumpe 23. Das Herausrutschen der Kanüle wird von der Überwachungsvorrichtung A erkannt, die mit der Blutbehandlungsvorrichtung B über eine drahtlose Verbindung, vorzugsweise eine Funkverbindung, kommuniziert. Hierfür verfügt die Blutbehandlungseinrichtung B über eine Datenübertragungseinheit 36, die eine Sendeeinheit 36A und eine Empfangseinheit 36B umfasst.

Das Auswertgerät 7 der Überwachungseinrichtung A weist eine Auswerteinheit 37 auf, die das Signal des Sensors 1 auswertet und ein für den Zustand des Gefäßzugangs charakteristisches Signal erzeugt, beispielsweise ein Signal, dass das Herausrutschen der Kanüle signalisiert.

Zur Herstellung der drahtlosen Verbindung mit der Datenübertragungseinheit 36 der Blutbehandlungsvorrichtung A weist das Auswertgerät 7 eine Datenübertragungseinheit 38 auf, die eine Sendeeinheit 38A und eine Empfangseinheit 38B umfasst. Beide Datenübertragungseinheiten 36, 38 bilden eine Schnittstelle, die nach dem Bluetooth-Standard arbeiten kann. Wenn die Kanüle 22 aus dem Gefäßzugang herausrutschen sollte und der Sensor 1 an der Punktionsstelle Blut erkennt, sendet die Sendeeinheit 38A des Auswertgerätes 7 ein Alarmsignal, das die Empfangseinheit 36B der Blutbehandlungsvorrichtung B empfängt. Daraufhin gibt die Alarmeinheit 33 der Blutbehandlungsvorrichtung B einen akustischen und/oder optischen Alarm und unterbricht die Blutbehandlung.

Darüber hinaus weist das Auswertgerät 7 eine Kontrolleinheit 39 und eine Signaleinheit 40 auf. Die Kontrolleinheit 39, die Bestandteil der Auswerteinheit 37 sein kann, überwacht die drahtlose Verbindung zwischen Auswertgerät und Blutbehandlungsvorrichtung. Die Signaleinheit 40 weist einen Vibrations-Signalgeber 40A auf. Derartige Signalgeber, die Vibrationen erzeugen, gehören zum Stand der Technik. Sie können einen Motor aufweisen, dessen Motorwelle eine Unwucht trägt. Der Vibrations-Signalgeber 40A ist mit der Vorder- und/oder Rückwand 10, 11 des Gehäuses 8 des Auswertgerätes 7 verbunden, so dass das gesamte Gehäuse in Schwingungen versetzt wird.

Bei dem Ausführungsbeispiel sendet die Sendeeinheit 36A der Blutbehandlungsvorrichtung B in bestimmten Zeitintervallen Bestätigungssignale, wenn die Empfangseinheit 36B der Blutbehandlungsvorrichtung Signale von der Sendeeinheit 38A des Auswertgerätes 7 empfängt. Die Kontrolleinheit 39 des Auswertgerätes 7 überwacht fortlaufend den Empfang der Bestätigungssignale. Wenn die Bestätigungssignale ausbleiben, weil die Funkverbindung gestört ist, erzeugt die Kontrolleinheit 39 ein Steuersignal, das die Signaleinheit 40 empfängt, so dass die Signaleinheit den Vibrations-Signalgeber 40A einschaltet. Die Kontrolleinheit 39 erzeugt bei dem Ausführungsbeispiel aber erst dann ein derartiges Steuersignal, wenn die drahtlose Verbindung für eine Zeitdauer unterbrochen ist, die größer als ein vorgegebener Zeitraum ist. Hierfür kann die Kontrolleinheit 40 über ein entsprechendes Zeitglied verfügen. Die von dem Zeitglied vorgegebene Zeitdauer sollte so bemessen sein, dass nur kurzzeitige Lageänderungen des Patienten nicht sofort zum Auslösen der Vibrationen führen.

Wenn sich der Patient auf das Auswertgerät 7 gelegt haben sollte, erkennt die Kontrolleinheit 40 sofort die Unterbrechung der drahtlosen Verbindung. Dann schaltet die Kontrolleinheit 40 den Vibrations-Signalgeber 40A der Signaleinheit 40 ein, der das Gehäuse zum Vibrieren bringt, was vom Patienten auch im Schlaf bemerkt wird. Der Patient kann das Auswertgerät 7 dann wieder freigeben, indem er eine andere Lage einnimmt, so dass die Verbindung wiederhergestellt ist.

Wenn die Verbindung wiederhergestellt ist, werden von der Blutbehandlungsvorrichtung wieder die Bestätigungssignale empfangen, wonach auf die Wiederherstellung der Verbindung geschlossen wird und das Vibrations-Signal automatisch ausgeschaltet wird. Sollte die Verbindung allerdings nicht wiederhergestellt oder wieder unterbrochen sein, wird das Vibrations-Signal nicht ausgeschaltet bzw. wieder eingeschaltet. An dem Auswertgerät kann auch ein Betätigungsorgan, beispielsweise ein Schalter oder Taster, zum Ausschalten des Vibrations-Signals vorgesehen sein.

Wenn das Auswertgerät Datentelegramme nur in bestimmten Zeitintervallen an die Blutbehandlungsvorrichtung senden sollte, wird das automatische Ausschalten des Vibrations-Signals nur mit einer gewissen zeitlichen Verzögerung erfolgen können. Dies kann dadurch vermieden werden, dass sofort nach Betätigung eines Betätigungsorgans durch den Patienten das nächste Datentelegramm an die Blutbehandlungsvorrichtung gesendet wird.

Es ist auch möglich, dass nach dem Erkennen einer Unterbrechung der drahtlosen Verbindung die Sendeeinheit 38A des Auswertgerätes 7 ein Signal an die Blutbehandlungsvorrichtung B sendet, so dass die Blutbehandlungsvorrichtung mit einer Signaleinheit ein akustisches und/oder optisches Signal gibt, das auch dem medizinischen Personal signalisiert, dass die Funkverbindung unterbrochen ist. Dieses akustische oder optische Signal wird aber allein nicht ausreichend sein, um den Patienten aus dem Schlaf aufzuwecken, was aber mit dem Vibrieren des Gehäuses 8 des Auswertgerätes 7 mit hoher Sicherheit erreicht wird, da der Patient erfahrungsgemäß empfindlich auf Vibrationen am Körper reagiert.

## Patentansprüche

1. Vorrichtung zur Überwachung eines Gefäßzugangs für eine extrakorporale Blutbehandlungsvorrichtung mit einem extrakorporalen Blutkreislauf, der eine arterielle Blutleitung mit einer arteriellen Kanüle und eine venöse Blutleitung mit einer venösen Kanüle aufweist, wobei die Vorrichtung zur Überwachung des Gefäßzugangs aufweist:
einen Sensor (1) zur Erfassung mindestens einer physikalischen Größe, die für den Zustand des Gefäßzugang charakteristisch ist,
ein mit dem Sensor (1) über ein Verbindungskabel (6) verbundenes Auswertgerät (7), das eine Auswerteinheit (37) zur Erzeugung von für den Zustand des Gefäßzugangs charakteristischen Signalen, insbesondere eines für einen fehlerhaften Gefäßzustand charakteristischen Signals aufweist, wobei das Auswertgerät (7) eine Datenübertragungseinheit (38) zur Herstellung einer drahtlosen Verbindung zwischen dem Auswertgerät und der Blutbehandlungsvorrichtung aufweist,
**dadurch gekennzeichnet, dass**
das Auswertgerät (7) eine Kontrolleinheit (39) aufweist, die derart konfiguriert ist, dass eine Unterbrechung der drahtlosen Verbindung zwischen dem Auswertgerät und der Blutbehandlungsvorrichtung erkannt wird, und eine Signaleinheit (40) aufweist, die ein Signal gibt, wenn die Kontrolleinheit (39) die Unterbrechung der drahtlosen Verbindung erkennt, wobei die Signaleinheit (40) einen Vibrations-Signalgeber (40A) aufweist, der die Unterbrechung der drahtlosen Verbindung mit Vibrationen signalisiert.

2. Vorrichtung nach Anspruch 1, **dadurch gekennzeichnet, dass** die Kontrolleinheit (39) derart konfiguriert ist, dass auf eine Unterbrechung der drahtlosen Verbindung zwischen dem Auswertgerät und der Blutbehandlungsvorrichtung erst dann geschlossen wird, wenn die drahtlose Verbindung für eine Zeitdauer unterbrochen ist, die länger als ein vorgegebener Zeitraum ist.

3. Vorrichtung nach Anspruch 2, **dadurch gekennzeichnet, dass** die Datenübertragungseinheit (38) eine Sendeeinheit (38A) zum Senden der für den Zustand des Gefäßzugangs charakteristischen Signale an die Blutbehandlungsvorrichtung und eine Empfangseinheit (38B) zum Empfangen von Bestätigungssignalen von der Blutbehandlungsvorrichtung aufweist, wobei die Kontrolleinheit (39) derart konfiguriert ist, dass die Kontrolleinheit erkennt, wenn die Empfangseinheit (38B) ein das Bestehen der drahtlosen Verbindung signalisierendes Bestätigungssignal der Blutbehandlungsvorrichtung nicht empfängt.

4. Vorrichtung nach einem der Ansprüche 1 bis 3, **dadurch gekennzeichnet, dass** die Datenübertragungseinheit (38) eine Funksignale oder optische Signale übertragende Einheit ist.

5. Vorrichtung nach einem der Ansprüche 1 bis 4, **dadurch gekennzeichnet, dass** der Sensor (1) als ein auf die Haut des Patienten aufzulegendes Pad aus einem flexiblen Material ausgebildet ist.

6. Vorrichtung nach einem der Ansprüche 1 bis 5, **dadurch gekennzeichnet, dass** der Sensor (1) als Sensor-Element einen Feuchtigkeits- oder Flüssigkeitssensor aufweist.

7. Vorrichtung nach einem der Ansprüche 1 bis 6, **dadurch gekennzeichnet, dass** das Auswertgerät (7) ein Gehäuse (8) aufweist, wobei der Vibrations-Signalgeber (8A) mit mindestens einer Gehäusewand (10, 11) verbunden ist.

8. Vorrichtung nach Anspruch 7, **dadurch gekennzeichnet, dass** das Gehäuse (8) des Auswertgerätes (7) als ein flaches Gehäuse mit einer Vorderwand (10) und einer Rückwand (11) und einer schmalen Seitenwand (13) ausgebildet ist, wobei der Vibrations-Signalgeber (40A) mit der Vorderwand (10) und/oder Rückwand (12) verbunden ist und das Verbindungskabel (6) an der Seitenwand (13) aus dem Gehäuse (8) herausgeführt ist.

9. Anordnung mit einer Vorrichtung (A) zur Überwachung eines Gefäßzugangs nach einem der Ansprüche 1 bis 8 und einer extrakorporalen Blutbehandlungsvorrichtung (B) mit einem extrakorporalen Blutkreislauf (I), der eine arterielle Blutleitung (20) mit einer arteriellen Kanüle (19) und eine venöse Blutleitung (21) mit einer venösen Kanüle (22) aufweist.

10. Anordnung nach Anspruch 9, **dadurch gekennzeichnet, dass** die extrakorporale Blutbehandlungsvorrichtung (A) eine Datenübertragungseinheit (36) zur Herstellung einer drahtlosen Verbindung zwischen der Blutbehandlungsvorrichtung (A) und dem Auswertgerät (7) aufweist.

11. Anordnung nach Anspruch 10, **dadurch gekennzeichnet, dass** die Datenübertragungseinheit (36) der Blutbehandlungsvorrichtung eine Sendeeinheit (36A) zum Senden von das Bestehen der drahtlosen Verbindung signalisierender Bestätigungssignale an das Auswertgerät (7) und eine Empfangseinheit (36B) zum Empfangen der für den Zustand des Gefäßzugangs charakteristischen Signale von dem Auswertgerät (7) aufweist.

12. Anordnung nach einem der Ansprüche 9 bis 11, **dadurch gekennzeichnet, dass** die Datenübertragungseinheit (36) der Blutbehandlungsvorrichtung (A) eine Funksignale oder optische Signale übertragende Einheit ist.

13. Anordnung nach einem der Ansprüche 9 bis 12, **dadurch gekennzeichnet, dass** die Blutbehandlungsvorrichtung (B) eine Alarmeinheit (33) aufweist, die derart ausgebildet ist, dass nach der Unterbrechung der drahtlosen Verbindung ein akustisches und/oder optisches Signal erzeugt wird.

14. Anordnung nach einem der Ansprüche 9 bis 13, **dadurch gekennzeichnet, dass** die Blutbehandlungsvorrichtung (B) eine Alarmeinheit (33) aufweist, die derart ausgebildet ist, dass nach dem Empfangen eines für einen fehlerhaften Gefäßzugang charakteristischen Signals ein akustischer und/oder optischer Alarm erzeugt wird.

15. Verfahren zur Überwachung eines Gefäßzugangs während einer extrakorporalen Blutbehandlung, bei dem mit einem Sensor mindestens eine physikalische Größe erfasst wird, die für den Zustand des Gefäßzugangs charakteristisch ist, und die für den Zustand des Gefäßzugangs charakteristische Größe in einem Auswertgerät ausgewertet wird, das über ein Verbindungskabel mit dem Sensor verbunden ist, wobei das Auswertgerät über eine drahtlose Verbindung ein für den Zustand des Gefäßzugangs charakteristisches Signal an eine Blutbehandlungsvorrichtung sendet, die das Signal des Auswertgerätes empfängt, **dadurch gekennzeichnet, dass** das Auswertgerät in Vibrationen versetzt wird, wenn die drahtlose Verbindung zwischen dem Auswertgerät und der Blutbehandlungsvorrichtung unterbrochen ist.

16. Verfahren nach Anspruch 15, **dadurch gekennzeichnet, dass** an der Blutbehandlungsvorrichtung ein akustisches und/oder optisches Signal wird, wenn die drahtlose Verbindung zwischen dem Auswertgerät und der Blutbehandlungsvorrichtung unterbrochen ist.

17. Verfahren nach Anspruch 15 oder 16, **dadurch gekennzeichnet, dass** an der Blutbehandlungsvorrichtung ein akustisches und/oder optisches Alarmsignal erzeugt wird, wenn von der Blutbehandlungsvorrichtung ein für einen fehlerhaften Gefäßzugang charakteristisches Signal empfangen wird, und/oder die Blutbehandlungsvorrichtung die Blutbehandlung unterbricht, wenn ein für einen fehlerhaften Gefäßzugang charakteristisches Signal empfangen wird.

18. Verfahren nach einem der Ansprüche 15 bis 17, **dadurch gekennzeichnet, dass** die drahtlose Verbindung eine Verbindung über Funksignale oder optische Signale ist.

## Claims

1. Device for monitoring a vascular access for an extracorporeal blood treatment device having an extracorporeal blood circuit which comprises an arterial blood line having an arterial cannula and a venous blood line having a venous cannula, the device for monitoring the vascular access comprising:
a sensor (1) for detecting at least one physical variable which is characteristic of the state of the vascular access,
an evaluation apparatus (7) which is connected to the sensor (1) via a connection cable (6) and comprises an evaluation unit (37) for generating signals which are characteristic of the state of the vascular access, in particular a signal which is characteristic of a faulty vascular state, the evaluation apparatus (7) comprising a data transmission unit (38) for producing a wireless connection between the evaluation apparatus and the blood treatment device,
**characterised in that**
the evaluation apparatus (7) comprises a supervision unit (39), which is configured to detect an interruption of the wireless connection between the evaluation apparatus and the blood treatment device, and comprises a signalling unit (40) which gives off a signal when the supervision unit (39) detects the interruption of the wireless connection, the signalling unit (40) comprising a vibration signal generator (40A) which uses vibrations to signal the interruption of the wireless connection.

2. Device according to claim 1, **characterised in that** the supervision unit (39) is configured such that a conclusion is only made regarding an interruption of the wireless connection between the evaluation apparatus and the blood treatment device when the wireless connection is interrupted for a period of time that is longer than a predetermined period of time.

3. Device according to claim 2, **characterised in that** the data transmission unit (38) comprises a transmitting unit (38A) for sending the signals which are characteristic of the state of the vascular access to the blood treatment device, and a receiving unit (38B) for receiving confirmation signals from the blood treatment device, the monitoring unit (39) being configured such that the monitoring unit detects when the receiving unit (38B) does not receive a confirmation signal from the blood treatment device signalling the existence of the wireless connection.

4. Device according to any of claims 1 to 3, **characterised in that** the data transmission unit (38) is a unit which transmits radio signals or optical signals.

5. Device according to any of claims 1 to 4, **characterised in that** the sensor (1) is designed as a pad which is made of flexible material and is to be placed on the patient's skin.

6. Device according to any of claims 1 to 5, **characterised in that** the sensor (1) comprises a moisture or liquid sensor as the sensor element.

7. Device according to any of claims 1 to 6, **characterised in that** the evaluation apparatus (7) comprises a housing (8), the vibration signal generator (8A) being connected to at least one housing wall (10, 11).

8. Device according to claim 7, **characterised in that** the housing (8) of the evaluation apparatus (7) is formed as a flat housing having a front wall (10) and a rear wall (11) and a narrow side wall (13), the vibration signal generator (40A) being connected to the front wall (10) and/or the rear wall (12) and the connection cable (6) being guided out of the housing (8) at the side wall (13).

9. Arrangement comprising a device (A) for monitoring a vascular access according to any of claims 1 to 8 and comprising an extracorporeal blood treatment device (B) having an extracorporeal blood circuit (I) which comprises an arterial blood line (20) having an arterial cannula (19) and a venous blood line (21) having a venous cannula (22).

10. Arrangement according to claim 9, **characterised in that** the extracorporeal blood treatment device (A) comprises a data transmission unit (36) for producing a wireless connection between the blood treatment device (A) and the evaluation apparatus (7).

11. Arrangement according to claim 10, **characterised in that** the data transmission unit (36) of the blood treatment device comprises a transmitting unit (36A) for sending confirmation signals to the evaluation apparatus (7) signalling the existence of the wireless connection and a receiving unit (36B) for receiving the signals which are characteristic of the state of the vascular access from the evaluation apparatus (7).

12. Arrangement according to any of claims 9 to 11, **characterised in that** the data transmission unit (36) of the blood treatment device (A) is a unit which transmits radio signals or optical signals.

13. Arrangement according to any of claims 9 to 12, **characterised in that** the blood treatment device (B) comprises an alarm unit (33) which is designed to generate an acoustic and/or optical signal after the wireless connection has been interrupted.

14. Arrangement according to any of claims 9 to 13, **characterised in that** the blood treatment device (B) comprises an alarm unit (33) which is designed to generate an acoustic and/or optical alarm following receipt of a signal which is characteristic of a faulty vascular access.

15. Method for monitoring a vascular access during extracorporeal blood treatment, in which at least one physical variable which is characteristic of the state of the vascular access is detected by a sensor, and the variable which is characteristic of the state of the vascular access is evaluated in an evaluation apparatus which is connected to the sensor via a connection cable, the evaluation apparatus sending, via a wireless connection, a signal which is characteristic of the state of the vascular access to a blood treatment device which receives the signal of the evaluation apparatus, **characterised in that** the evaluation apparatus is made to vibrate when the wireless connection between the evaluation apparatus and the blood treatment device is interrupted.

16. Method according to claim 15, **characterised in that** an acoustic and/or optical signal is generated at the blood treatment device when the wireless connection between the evaluation apparatus and the blood treatment device is interrupted.

17. Method according to either claim 15 or claim 16, **characterised in that** an acoustic and/or optical alarm signal is generated at the blood treatment device when a signal which is characteristic of a faulty vascular access is received by the blood treatment device, and/or the blood treatment device interrupts the blood treatment when a signal which is characteristic of a faulty vascular access is received.

18. Method according to any of claims 15 to 17, **characterised in that** the wireless connection is a connection via radio signals or optical signals.

## Revendications

1. Dispositif servant à surveiller un accès vasculaire pour un dispositif de traitement du sang extracorporel avec un circuit sanguin extracorporel, qui présente un conduit sanguin artériel avec une canule artérielle et un conduit sanguin veineux avec une canule veineuse, dans lequel le dispositif servant à surveiller l'accès vasculaire présente :
un capteur (1) servant à détecter au moins une grandeur physique, qui est caractéristique de l'état de l'accès vasculaire,
un appareil d'analyse (7) relié au capteur (1) par l'intermédiaire d'un câble de liaison (6), qui présente une unité d'analyse (37) servant à produire des signaux caractéristiques de l'état de l'accès vasculaire, en particulier un signal caractéristique d'un accès vasculaire défectueux, dans lequel l'appareil d'analyse (7) présente une unité de transmission de données (38) servant à établir une liaison sans fil entre l'appareil d'analyse et le dispositif de traitement du sang,
**caractérisé en ce que**
l'appareil d'analyse (7) présente une unité de contrôle (39), qui est configurée de telle manière qu'une coupure de la liaison sans fil entre l'appareil d'analyse et le dispositif de traitement du sang est identifiée, et présente une unité de signal (40), qui émet un signal, quand l'unité de contrôle (39) identifie la coupure de la liaison sans fil, dans lequel l'unité de signal (40) présente un émetteur de signal à vibrations (40A), qui signale la coupure de la liaison sans fil avec des vibrations.

2. Dispositif selon la revendication 1, **caractérisé en ce que** l'unité de contrôle (39) est configurée de manière à conclure à une coupure de la liaison sans fil entre l'appareil d'analyse et le dispositif de traitement du sang seulement quand la liaison sans fil est coupée pour une durée qui est plus longue qu'une période spécifiée.

3. Dispositif selon la revendication 2, **caractérisé en ce que** l'unité de transmission de données (38) présente une unité d'envoi (38A) servant à envoyer les signaux caractéristiques de l'état de l'accès vasculaire au dispositif de traitement du sang et une unité de réception (38B) servant à recevoir du dispositif de traitement du sang des signaux d'actionnement, dans lequel l'unité de contrôle (39) est configurée de telle manière que l'unité de contrôle identifie quand l'unité de réception (38B) ne reçoit pas un signal d'actionnement, signalant l'existence de la liaison sans fil, du dispositif de traitement du sang.

4. Dispositif selon l'une quelconque des revendications 1 à 3, **caractérisé en ce que** l'unité de transmission de données (38) est une unité transmettant des signaux radio ou des signaux optiques.

5. Dispositif selon l'une quelconque des revendications 1 à 4, **caractérisé en ce que** le capteur (1) est réalisé en tant que tampon à placer sur la peau du patient, composé d'un matériau flexible.

6. Dispositif selon l'une quelconque des revendications 1 à 5, **caractérisé en ce que** le capteur (1) présente, en tant qu'élément de capteur, un capteur d'humidité ou de liquide.

7. Dispositif selon l'une quelconque des revendications 1 à 6, **caractérisé en ce que** l'appareil d'analyse (7) présente un boîtier (8), dans lequel l'émetteur de signaux à vibrations (8A) est relié à au moins une paroi de boîtier (10, 11).

8. Dispositif selon la revendication 7, **caractérisé en ce que** le boîtier (8) de l'appareil d'analyse (7) est réalisé en tant que boîtier plat avec une paroi avant (10) et une paroi arrière (11) et une paroi latérale (13) étroite, dans lequel l'émetteur de signaux à vibrations (40A) est relié à la paroi avant (10) et/ou à la paroi arrière (12) et le câble de liaison (6) est guidé au niveau de la paroi latérale (13) pour sortir du boîtier (8).

9. Ensemble avec un dispositif (A) servant à surveiller un accès vasculaire selon l'une quelconque des revendications 1 à 8 et un dispositif de traitement du sang (B) extracorporel avec un circuit sanguin (I) extracorporel, qui présente un conduit sanguin artériel (20) avec une canule artérielle (19) et un conduit sanguin veineux (21) avec une canule veineuse (22).

10. Ensemble selon la revendication 9, **caractérisé en ce que** le dispositif de traitement du sang (A) extracorporel présente une unité de transmission de données (36) servant à établir une liaison sans fil entre le dispositif de traitement du sang (A) et l'appareil d'analyse (7).

11. Ensemble selon la revendication 10, **caractérisé en ce que** l'unité de traitement de données (36) du dispositif de traitement du sang présente une unité d'envoi (36A) servant à envoyer à l'appareil d'analyse (7) des signaux d'actionnement signalant l'existence de la liaison sans fil et une unité de réception (36B) servant à recevoir de l'appareil d'analyse (7) les signaux caractéristiques de l'état de l'accès vasculaire.

12. Ensemble selon l'une quelconque des revendications 9 à 11, **caractérisé en ce que** l'unité de transmission de données (36) du dispositif de traitement du sang (A) est une unité transmettant des signaux radio ou des signaux optiques.

13. Ensemble selon l'une quelconque des revendications 9 à 12, **caractérisé en ce que** le dispositif de traitement du sang (B) présente une unité d'alarme (33), qui est réalisée de telle manière qu'après la coupure de la liaison sans fil, un signal acoustique et/ou optique est produit.

14. Ensemble selon l'une quelconque des revendications 9 à 13, **caractérisé en ce que** le dispositif de traitement du sang (B) présente une unité d'alarme (33), qui est réalisée de telle manière qu'après la réception d'un signal caractéristique d'un accès vasculaire défectueux, une alarme acoustique et/ou optique est produite.

15. Procédé servant à surveiller un accès vasculaire pendant un traitement du sang extracorporel, où au moins une grandeur physique est détectée avec un capteur, qui est caractéristique de l'état de l'accès vasculaire, et la grandeur caractéristique de l'état de l'accès vasculaire est analysée dans un appareil d'analyse, qui est raccordé au capteur par l'intermédiaire d'un câble de liaison, dans lequel l'appareil d'analyse envoie par l'intermédiaire d'une liaison sans fil un signal caractéristique de l'état de l'accès vasculaire au dispositif de traitement du sang, qui reçoit le signal de l'appareil d'analyse, **caractérisé en ce que** l'appareil d'analyse est mis en vibration quand la liaison sans fil entre l'appareil d'analyse et le dispositif de traitement du sang est coupée.

16. Procédé selon la revendication 15, **caractérisé en ce qu'**un signal acoustique et/ou optique est envoyé au dispositif de traitement du sang quand la liaison sans fil entre l'appareil d'analyse et le dispositif de traitement du sang est coupée.

17. Procédé selon la revendication 15 ou 16, **caractérisé en ce qu'**un signal d'alarme acoustique et/ou optique est produit au niveau du dispositif de traitement du sang quand un signal caractéristique d'un accès vasculaire défectueux est reçu par le dispositif de traitement du sang, et/ou le dispositif de traitement du sang coupe le traitement du sang quand un signal caractéristique d'un accès vasculaire défectueux est reçu.

18. Procédé selon l'une quelconque des revendications 15 à 17, **caractérisé en ce que** la liaison sans fil est une liaison par l'intermédiaire de signaux radio ou de signaux optiques.
